# EUROPEAN PATENT SPECIFICATION

(11) **EP 2 954 886 B1**
(45) Date of publication and mention of the grant of the patent: **28.08.2019**
(21) Application number: 14305891.5
(22) Date of filing: 12.06.2014
(51) Int. Cl.: A61K 9/12, A61K 9/00, A61K 47/34, A61K 31/20, A61K 31/201, A61K 31/202

(54) **Pheromonal composite foam compositions**
Pheromonalverbundschaumstoffzusammensetzungen
Compositions phéromonales de mousse composite

(43) Date of publication of application: 16.12.2015
(73) Proprietor: Ceva Santé Animale, 33500 Libourne Cedex (FR)
(72) Inventor: Beck, Alexandra, 33910 SAVIGNAC DE L'ISLE (FR)
(74) Representative: Tezier Herman, Béatrice

(56) References cited:
- WO-A1-01/74582
- WO-A2-2008/152444
- DD-A1- 277 385
- US-A1- 2011 150 822
- US-B1- 6 500 862
- SHARP J L ET AL: "LONGEVITY AND ATTRACTIVITY TO MALE LESSER PEACHTREE BORER SYNANTHEDON-PICTIPES OF E Z-3 13 OCTA DECA DIEN-1-OL ACETATE EVAPORATED FROM VARIOUS DISPENSERS", ENVIRONMENTAL ENTOMOLOGY, ENTOMOLOGICAL SOCIETY OF AMERICA, COLLEGE PARK, MD, US , vol. 9, no. 6 1 December 1980 (1980-12-01), pages 818-820, XP008173813, ISSN: 0046-225X Retrieved from the Internet: URL:http://www.ingentaconnect.com/content/ esa/envent/1980/00000009/00000006/art00021

## Description

The present invention relates to a composite comprising a composition of pheromones and a composite foam, said composite can be for a use to prevent and / or treat at least one medical or behavioral stress problem of a non-human mammal. It also relates to a device comprising said composite, and to a kit comprising the device.

### BACKGROUND OF THE INVENTION

Pheromone therapy, or 'pheromonatherapy', is a great way of managing non-human mammals welfare and more particularly some common stress-related behavioral disorders in domestic mammals, such as dogs and cats, without the need for drugs and their associated side-effects.

Pheromones are chemical substances used for communication between individuals of the same species. The precise mechanism of action of most pheromones is still unknown but they induce some modifications in both the limbic system and the hypothalamus and seems to be detected by the vomeronasal organ (VMO), which is part of the accessory olfaction system (P.Pageat and al. "Current research in canine and feline pheromones" Vet. Clin. Small Anim. - 33 (2003) 187-211).

Pheromonotherapy has however some specific technical problems:
i) In natural conditions, the pheromones are not expelled alone. The emitting animal shows a particular posture or mimicry (e.g., the posture for urine marking in cats), sometimes shows a part of its body that is usually hidden (eg, the anal area), modifies the marked substrate (e.g., scratches on the ground in dogs or on vertical surfaces in cats), and also expels some individual odors and the pheromone itself. The function of all these messages is to induce the opening of the VNO, which is usually closed.
ii) The appropriate pheromone has to be emitted at the right time and on the right location so as to obtain the expected behavioral results.

Pheromonotherapy is successfully used to prevent and / or treat at least one medical or behavioral stress problem of a non-human mammal for pets and especially for cats and dogs.

For cats, Feliway® (Ceva Santé Animale) was the first synthetic version of naturally occurring feline facial pheromones in a spray form and the first pheromonotherapy used for cat to prevent and / or treat at least one medical or behavioral stress problem. The product mimics the feline's facial pheromones and can be used to calm the pet in stressful situations. When sprayed onto objects in the cat's environment, Feliway® decreases the cat's arousal and stops urine marking and vertical scratching, and other stress-related behaviours. The user always needs to wait for about 15 minutes before letting the cat near any Feliway® sprayed areas, in particular as to let the volatile carrier solvent evaporate.

As this spray schedule may be challenging to comply with, convenient plug-in Feliway® devices have been developed and commercialized by Ceva Santé Animale. These plug-in offer a no-hassle way for pet owners to calm their cats. The pet owner has just to attach the Feliway® source to the plug-in diffuser, plug it into an electric socket in the room most used by the cat, and continue use for four weeks with a liquid plug in diffuser or one week with a cartridge diffuser (Catitude®, Ceva Santé Animale) to ensure no relapse in marking behavior.

For dogs, dog appeasing pheromone (DAP® or Adaptil®; Ceva Sante Animale) is a synthetic analog of natural dog-appeasing pheromone that has been promoted as an adjunct treatment to improve conditions such as separation-related behavior problems, loud noise phobias, stress and anxiety. Dog-appeasing pheromone has been reported to reduce separation-induced anxiety, fear in puppies in a new environment and anxiety and stress during transportation. It can also decrease the stress of dogs in public shelter, the training-derived stress of working dogs (police dogs, guide dogs) and the anxiety of puppies in learning and socialization processes. Also, Adaptil® might be a potential treatment for dogs that are fearful of fireworks. Use of Adaptil® in veterinary clinics was significantly associated with greater relaxation in dogs, but did not have an effect on aggression.

Adaptil® is available in two main forms: spray and plug in diffuser.
Adaptil® spray must be sprayed 15 minutes before the dog is introduced into the environment (car, carrier, kennel, etc..), in particular to get the volatile carrier solvent evaporated. Effects should last approximately 4-5 hours and repeated applications can be necessary if the dog owner notice a reduced effect. It is recommended not to spray Adaptil® directly on animals nor near an animal's face. Adaptil® diffuser can be plugged into an electric socket in a room, frequently used by the dog, and continually used for four weeks with a liquid plug in diffuser to comfort both puppies and adult dogs.

US 6,500,862 B1 discloses microemulsions for the administration of fatty acids as pheromones to humans or animals.

However the prior art does not provide so far an easy and convenient method for diffusion of pheromones.

There is a need for new pheromonotherapy methods where a convenient and efficient pheromones diffusion on the non-human mammals is obtained, for instance, when cats and dogs travel in cars, trains or planes, visit vets, or there are fireworks or any noise or light inducing stress and anxiety to animals and resulting in harmful or annoying behaviors..

### SUMMARY OF THE INVENTION

This present invention relates to a composite comprising a composition of pheromones and a composite foam, wherein the pheromones are selected from monocarboxylic and dicarboxylic acids with hydrocarbonated chains, saturated or unsaturated, linear or branched, liable to treat behavior or medical changes related to anxiety or stress of non-human mammals, and/or esters or salts of said acids, and the composite foam is a polyurethane that comprises both hydrophobic and hydrophilic polyurethane foam entities.

The composite according to the invention is suitable for a use to prevent and / or treat at least one medical or behavioral stress problem of a non-human mammal, in particular by administration of the pheromones based composition to said mammal. Consequently, a further object of the invention is the composite as defined above for a use to prevent and/or treat at least one medical or behavioral stress problem of a non-human mammal.

Another object of the present invention is a device comprising a composite comprising a composition of pheromones and a composite foam, both as defined in the present invention, the device being more specifically suitable for administration of the pheromones onto the face of a non-human mammal. The said device preferably comprises means to spray the said composition.

The said device may be, for example, a container, such as a bottle, with a spraying means. A kit comprising a device may also be envisaged, the said kit comprising a device, in particular a container, a composite as defined in the present invention, and optionally a booklet giving instructions to use said device. The said device may be in ready-to-use form, i.e. prefilled, or may need to be filled before use. In the latter case, the composition comprising pheromones and the composite foam may be in separate devices (such as vials).

### DETAILED DESCRIPTION

In contrast with conventional pheromonotherapy methods, the method according to the present invention allows efficient pheromones effects preventing and / or treating thereby at least one medical or behavioral stress problem of a non-human mammal, in particular due to a convenient and easy diffusion of pheromones using composite foam.

The composite according to the invention comprises a composition of pheromones, which are fatty acids or derivatives thereof as defined in the claims, which is either in the forms of dilutions or emulsions in solvents or in surfactants or not, and a composite foam. According to the specific embodiment where the composition of pheromones is not in the forms of dilutions or emulsions in solvents or in surfactants, the composition is called herein a mixture of "pure pheromones", since the mixture consists essentially of pheromones. According to a specific embodiment, the composition of pheromones can be free of volatile solvent, such as alcohol (e.g. ethanol).

The composition can be administered directly onto the face of a non-human mammal, or some drops thereof can be applied on the face of said mammal, or can be spread in a place where the mammal is located, giving rise to a satisfactory result; the general behavior of the treated non-human mammal is significantly improved. Therefore, medical or behavioral stress problems of non-human mammals are significantly reduced.

The term « pheromone » as used herein refers to any compound that mimics certain naturally occurring pheromones that have a calming effect on mammals or any naturally occurring pheromones, said naturally occurring pheromones are one or more secreted compounds by an organism of a specific species which entails a foreseeable reaction (such as for example a calming effect) to another mammal of the same species or another species. More preferably, pheromones correspond to at least one fatty acid or derivative thereof, more specifically ester derivative, such as methyl ester.

The present invention deals with a composition or mixture comprising one or more pheromones, which are fatty acids or derivatives thereof, and/or ester or methyl ester derivatives thereof, as active ingredients. The composition or mixture thus may comprise or consist essentially of a pure fraction or mixture of pheromones which may be administered by by propelling or spraying onto the face of non-human mammals, by applying on the face some drops of said composition or mixture, or by spreading in a place where the mammal is located. According to a specific embodiment where the composition of pheromones is a mixture of pheromones, the proportions of pheromones, or more specifically fatty acid, ester derivatives or methyl ester derivatives, are approximately between 77 and 94%, preferably between 85 and 90% or equal to approximately 90% by weight with respect to the total weight of the mixture.

According to another specific embodiment where the composition of pheromones is a mixture of pheromones in association with a physiologically acceptable carrier or support, the proportions of pheromones, more specifically fatty acid, ester derivatives or methyl ester derivatives, are approximately between 10 and 80%, preferably from 30 and 60%, more preferably from 35 to 45%, by weight with respect to the total weight of the composition.

The term « fatty acid », according to the invention, refers to monocarboxylic and dicarboxylic acids with hydrocarbonated chains, saturated or unsaturated, linear or branched, liable to treat behavior or medical changes related to anxiety or stress of non-human mammals. The fatty acids preferably have from 3 to 22 carbon atoms. They can be selected for instance from propanoic acid (propionic), butanoic acid (butyric), pentanoic acid (valeric), hexanoic acid (caproic), heptanoic acid (enanthic), octanoic acid (caprylic), nonanoic acid, decanoic acid (capric), undecanoic acid (undecylic), dodecanoic acid (lauric), tridecanoic acid (tridecylic), pentadecanoic acid, heptadecanoic acid (margaric), octadecanoic acid (stearic), eicosanoic acid (arachidic), arachidonic acid, heneicosanoic acid (heneicosylic), tricosanoic (tricosylic), tetracosanoic acid (lignoceric), pentacosanoic acid (pentacosylic), hexacosanoic acid (cerotic), heptacosanoic acid (heptacosylic), octacosanoic acid (montanic), nonacosanoic acid (nonacosylic), triacontanoic acid (melissic), henatriacontanoic acid (henatriacontylic), dotriacontanoic acid (lacceroic), tritriacontanoic acid (psyllic), tetratriacontanoic acid (geddic), pentatriacontanoic acid (ceroplastic), hexatriacontanoic acid (hexatriacontylic), nonanedioic acid (azelaic), oleic acid, γ-linoleic acid, palmitic acid, palmitoleic acid, linoleic acid, n-butyric acid, isobutyric acid, α -methylbutyric acid, tetradecanoic acid (myristic), pentadecylic acid (n-pentadecanoic) and heptanedioic acid (pimelic). "Derivatives of fatty acids" means all active, volatile derivatives of fatty acids. Derivatives according to the invention are the ester or methyl ester or salt forms.

According to a specific embodiment, they are chosen among oleic acid, palmitic acid, azelaic acid, pimelic acid, capric acid, myristic acid, palmitoleic acid, linoleic acid, linolenic acid, stearic acid, valeric acid, arachidonic acid, lauric acid, n-butyric acid, isobutyric acid, α-methylbutyric acid, pivalic acid, γ-linoleic acid, eicosapentanoic acid, pentadecanonic acid, tridecanoic acid, docosahexanoic acid, and a mixture thereof.

The compositions or mixtures may comprise a pure fraction of at least one fatty acid such as oleic acid, linoleic acid, palmitic acid, a derivative thereof, ester or methyl ester derivative thereof, as defined in the claims. The mixtures may also comprise without limitations, compositions of fatty acids that are suitable to reduce stress and anxiety behaviors of non-human mammals, derivatives thereof, esters or methyl ester derivatives thereof, as defined in the claims. By way of examples, we can quote fatty acid mixtures, or their derivatives, ester or methyl ester derivatives, such as:
a mixture of oleic and palmitic acid;
a mixture of oleic and n-butyric acid;
a mixture of oleic acid, palmitic acid and linoleic acid;
a mixture of oleic acid, palmitic acid, linoleic acid and palmitoleic acid;
a mixture of capric acid, lauric acid, myristic acid, palmitoleic acid, palmitic acid, linoleic acid and oleic acid;
a mixture of oleic acid, palmitic acid, linoleic acid, myristic acid;
a mixture of oleic acid, palmitic acid, linoleic acid, lauric acid and myristic acid
a mixture of oleic acid, palmitic acid, linoleic acid, myristic acid and pentadecanonic acid;
a mixture of oleic acid, palmitic acid, linoleic acid, myristic acid, pentadecanonic acid and stearic acid;
a mixture of oleic acid, palmitic acid, linoleic acid, myristic acid, lauric acid and pentadecanonic acid;
a mixture of lauric acid, myristic acid, pentadecanonic, palmitic acid, stearic acid, oleic acid, linoleic acid; or
a mixture of oleic acid, azelaic acid, pimelic acid and palmitic acid.

The mixtures may comprise the above-mentioned fatty acids in appropriate proportions which are well known to any skilled person. By way of example, the mixtures can contain:
about 55-65% of oleic acid and 45-35% of palmitic acid, their derivatives, and its ester or methyl ester derivatives;
about 45% of oleic acid, 16% of azelaic acid, 18% of pimelic acid, and 21% of palmitic acid, their derivatives, ester or methyl ester derivatives thereof;
about 30% of palmitic acid, 30% of oleic acid, and 40% linoleic acid, their derivatives, ester and methyl ester derivatives thereof; or
about 30% of palmitic acid, 40% of linoleic acid, 10% acid palmitoleic and 20% of oleic acid, their derivatives, ester and methyl ester derivatives thereof; or
about 30% of oleic acid, 20% of palmitic acid, 20% of linoleic acid, 10% stearic acid, 5% pentadecanoic acid, 5% myristic acid and 2 % lauric acid, their derivatives, ester and methyl ester derivatives thereof.

According to an embodiment of the present invention, the fatty acid mixture comprises a mixture of appeasing fatty acids for cats. Preferably, this mixture comprises at least a therapeutically effective amount of active fatty acids, derivatives thereof, ester or methyl ester derivatives thereof and preferably chosen among oleic acid, azelaic acid, pimelic acid and palmitic acid. The pure mixture of fatty acids comprises at least between about 45-60% oleic acid, 6-10% of azelaic acid, 8-12% of pimelic acid, and 13-18% of palmitic acid. In the case where ester and methyl ester forms are used, the pure mixture comprises between about 45-65% methyl oleate, between about 6-10% dimethyl azelate, between about 8-12% of dimethyl pimelate, and between about 13-18% of methyl palmitate. Preferably, the pure mixture of esters of fatty acids consists approximately 47-51% of methyl oleate, approximately 7-9% of dimethyl azelate, between 9-11% of dimethyl pimilate and approximately 14-16% of methyl palmitate.

According to another embodiment of the invention, the mixture comprises a mixture of fatty acids that are appeasing for dogs. Preferably, this mixture comprises a therapeutically effective amount of fatty acids, their derivatives, ester or methyl ester derivatives thereof. These are preferably chosen among lauric acid, myristic acid, pentadecanoic acid, palmitic acid, stearic acid, oleic acid and linoleic acid. By way of example, a pure mixture of ester fatty acids for dogs comprises about 35% of methyl oleate, about 2% of methyl laurate, about 13% methyl stearate, about 21% methyl linoleate, about 5% methyl myristate, about 4% methyl pentadecanoate, and about 20% methyl palmitate.

They are more specifically devoid of solvent, excipients, vegetable extracts, such as vegetable extracts of *Valeriana* or *Nepeta* (more specifically *Nepeta cataria*), or even aqueous phase. According to a specific embodiment, they only comprise one oily phase corresponding to a pure fraction of fatty acids, their derivatives, ester or methyl ester derivatives thereof and are not in the form of emulsions or micro-emulsions. They are present in the form of oily and liquid phases.

Unless otherwise specified, the percentages are expressed by weight.

The composite foam comprised in the composite according to the invention is more specifically polyurethane foam. The preferred composite foam used in the present invention is more specifically a composite foam as described in WO200174582. In accordance with the invention, the foam is a composite foam which has properties of both conventional hydrophobic and hydrophilic polyurethane foams. More specifically, the composite foam comprises both hydrophobic and hydrophilic polyurethane foam entities. The composite foam can comprise open cell hydrophobic polyurethane foams and open cell hydrophilic foams. The cells in the hydrophobic foams may vary in their degree of openness. Hydrophobic foams useful in the practice of the invention include those having a reticulated or substantially reticulated cell structure as well as those having an open-cell non-reticulated structure.

More specifically, the composite foam includes a hydrophobic scaffold foam, such as an open cell or open cell reticulated polyurethane foam, coated with an open cell hydrophilic polyurethane foam. This is accomplished by coating the inside surface of the open cell foam with a polyurethane prepolymer emulsion and allowing the composite to cure. What results is a composite foam that uses the open cell polyurethane foam as a scaffold or a substrate on which the hydrophilic polyurethane foam is cast.

In another aspect of the present invention, the composite foam is formed by contacting a reticulated hydrophobic polyurethane foam with a solution of a hydrophilic polyurethane prepolymer in a solvent such as acetone and the like. The solvent of the polyurethane prepolymer solution is subsequently recovered thus coating the reticulated hydrophobic polyurethane foam with the hydrophilic polyurethane prepolymer.

In another embodiment of the invention the composite foam is provided by contacting the hydrophobic polyurethane foam with a liquid phase of a hydrophilic polyurethane prepolymer at temperatures sufficient to lower the viscosity and thereby control the coating weight of the hydrophilic prepolymer.

Specifically, the hydrophilic coating provides for the hydrophilic character, while the reticulated foam provides for physical strength and the good flow-through aspects that characterize a reticulated foam. Thus, while the hydrophilic coating will swell when it absorbs water, the reticulated foam is sufficiently strong to prevent an increase in the size of the composite foam.

It is a further aspect of the invention to provide a composite for a use for the controlled release of a component into a stream of fluid passing through the composite foam.

The stream can be a gas or liquid, but in either case the action of the composite foam is to release into the stream the component resident and more particularly in the hydrophilic polyurethane foam coating.

According to the present invention, the component resident is the pheromone based composition.

The composite according to the invention can be prepared easily by contacting the composite foam and the pheromone based composition, both as defined in the present invention. Generally, the pheromone based composition is in a liquid form (from oily to liquid phases) and can impregnate the composite foam.

The composite according to the invention is suitable for a use to prevent and / or treat of at least one medical or behavioral stress problem of a non-human mammal, in particular by administering the pheromones based composition to said non-human mammal. More specifically, the composite comprises the composite foam as defined above impregnated with the pheromone based composition. The pheromone based composition can thus be delivered by pressing the composite foam impregnated with said composition. More specifically, the composition of pheromones according to the invention is administered to a non-human mammal by propelling the pheromone based composition by a stream of fluid passing through the composite foam impregnated with the said composition. More preferably, the stream of fluid is a gas and especially air.

The composition of pheromones according to the invention from the composite can be administered to a non-human mammal by propelling the composition onto, over or nearby the face of a non-human animal or a part thereof. The method of administration of the composition according to the invention can be implemented directly in a place where a non-human mammal is located. The place can be of any kind such as for example a confined place, for instance a car, truck, carrier, small house or kennel.

The administration onto the face of a non-human mammal or a part thereof can be carried out by propelling, spraying, applying or diffusion of the composition of pheromones from the composite. The composition of pheromones can be propelled near the face, more specifically the distance between the propelling location and the face of the animal can be for instance less than or equal to 300, 200, 100, or 50 cm, and more preferably is less than or equal to 40 cm, 30, 20, 10 or 5 cm.

The administration onto the face of non-human mammals according to the invention can thus be carried out easily and anywhere.

In many mammals, pheromones are thought to be detected essentially by the vomeronasal organ (VNO or Jacobson's organ). The vomeronasal organ is also known as the accessory olfactory organ. It is an olfactory sense organ that is found in most animals. It is positioned at the base of the nasal cavity, within the roof of the mouth, and is separated into two parts by the nasal septum. It is situated close to the vomer and nasal bones, hence the name vomeronasal organ. It is contained within a bony capsule which opens into the base of the nasal cavity, and is lined with olfactory mucosa. The vomeronasal organ contains sensory chemoreceptors, and is involved in the first processing step of the accessory olfactory system. This feature allows pheromones present even in extremely small quantities to very selectively trigger certain biochemical processes in the animal and to have appeasing effects both rapid and effective.

Thus, the composite comprising composition of pheromones according to the invention can be administered to a non-human mammal by propelling of the composition onto, over or nearby the face of said mammal or a part thereof, and more specifically onto, over or nearby the vomeronasal organ, nose, mouth, or a part thereof.

According to another specific embodiment, the composition of pheromones can also be directly administrated on the face of the non-human animal or a part thereof. According to this embodiment, the composite according to this embodiment can thus be applied as a stick on the face of the animal or a part thereof, such as nose, mouth or vomeronasal organ.

According to another specific embodiment, the composition of pheromones according to the invention can also be released from the composite into the ambient air in the non-human animals' immediate environment.

It is a further aspect of the invention to provide a composite for a use for the controlled release of pheromones into a stream of fluid passing through the composite foam impregnated with the composition of pheromones. The stream can be a gas or liquid, but in either case the action of the composite foam is to release pheromones with the stream.

"Non-human mammals" is intended to mean all animals with the exception of human. The non-human animals include domestic, farm, and zoo animals, including cats, dogs, horses, cattle, tigers, lions, bears, elephants, pigs, boars, etc.. According to the present description, when cats or dogs are cited by way of example, it can be generalized to any other domestic, farm, and zoo animals. More specifically, it refers to cats and dogs.

The amount of pheromones to be administered according to the invention varies and depends for instance on the nature of pheromones and the non-human mammal but it should be sufficient to prevent and / or treat of at least one medical or behavioral stress problem of a non-human mammals. In a specific embodiment, the effect should be obtained immediately, e.g. within less than 10 minutes, more preferably less than 5 minutes. The administration can be renewed, one, two or three times, if no satisfactory effect is obtained or if reduced effect is observed.

The composition of pheromones as defined above is particularly effective to prevent and / or treat at least one medical or behavioral stress problem of a non-human mammal.

More particularly, the composition of pheromones according to the present invention can prevent territorial urinary marking and/or reduce anxiety or stress related symptoms, and/or familiarize the animals with a new environment, and/or prevent noisy outbursts, soiling, destructions, stamping in the territories or aggressiveness. The composition of pheromones is equally useful for the prevention and/or treatment of recurrent idiopathic cystitis, notably in stressed or anxious felines.

The administration of the composition of pheromones may take place over very short periods of time such as for example during traveling periods. It can be renewed if necessary in order to be adapted to stress and anxiety behaviors of the non-human mammals, according to the severity of the pathology.

The present invention also relates to the use of composition of pheromones, for the preparation of a composite comprising the composition of pheromones as defined above and composite foam, more specifically a polyurethane foam, as described herein, to prevent and / or treat at least one medical or behavioral stress problem of a non-human mammals.

It also relates to a method to prevent and / or treat at least one medical or behavioral stress problem of a non-human mammal which comprises administering to the said animal an effective amount of the composition of pheromones as described above (or pheromones comprised therein), wherein said composition (or pheromones) is released (are released) from the composite of the invention.

The novel modes of administration and dosages according to the present invention are particularly efficient on canine and feline and more especially on dogs and cats.

Behavior stress origins for dogs today could be found for example in the owners' hectic lifestyles difficult to cope with, because owners are constantly on the go whether it be moving house, going on holiday, having parties and even loud noises people make for fun like fireworks night. Accordingly, medical or behavior stress signs increases such as for example excessive barking, agitation, trembling, shaking, shivering, drooling/salivating, cowering and hiding, licking, scratching, yawning, clinging to owners, trying to run away/attempting to escape, soiling the place, excessive pacing and panting, refusing to eat, vomiting, whining, urinating/defaecating, or any combination of said signs.

Cats, unlike dogs, are considered to be territorial animals. They have very specific behaviors centralized around their territory. Only when their environment is secure will some cats develop a relationship with other pets or humans. Stress origins for cats can be for example multi-cat households, visiting the vets, moving home, new house furniture and redecorating, cat carrier... The medical or behavior stress signs in cats can be illustrated for example by scratching, urine marking, aggression, food intake disorders (anorexia or over-feeding), overgrooming (bald areas) or undergrooming (matted or soiled fur), house soiling, decreased levels of activity, appearing withdrawn (reduced desire to play or interact to owners), cystitis, dermatological problems including excessive licking, alopecia, anorexia, obesity, diarrhea, infections tract, or any combination of said signs.

The composites, compositions, mixtures, uses and methods according to the present invention thus allow preventing and / or treating at least one medical or behavioral stress problem of a non-human mammal. For example, repetitive urinary markings of cats occurring in case of stress and / or anxiety of cats due to circumstances or particular changes to their immediate environment. It also allows the improvement of the conditions for the non-human mammals to familiarize themselves with their new environment, especially during travels, and / or to prevent stamping and territorial destructions, and/or reduce noisy outbursts. The composites, compositions, and mixtures according to the present invention are thus intended to be used in circumstances where, for instance, a dog has shown, or is anticipated to show, signs of stress and anxiety when being transported in a car.

For cats, the beneficial effects can be measured for example by the reduction of the urinary markings and by the repeated physical contact or rubbing against new objects and people that surround them. On the whole, the composition of pheromones according to the present invention allows the improvement of behavior of the non-human mammals in their environment and people present in that environment, with a notable reduction of stress, anxiety, urinary marking and or noisy outbursts, as well as behavior that is non aggressive and more relaxed and more affectionate, especially with their owners.

The composition of pheromones according to the invention may be administered by any means or devices suitable for administration in ambient temperature of said composition onto the face of a non-human mammal or a part thereof.

The present invention also deals with a device comprising a composite of the invention, preferably said device presents means for the controlled release of the composition of pheromones into a stream of fluid passing through the composite foam containing the composition of pheromones, and preferably the said fluid is a gas or a liquid. The said device may comprise means to propel, diffuse or spray the said composition. Generally, according to the invention, there is a stream of fluid passing through the composite foam impregnated with the said composition of pheromone. There is no need to help diffusion of pheromones by heat. The device can be or comprise any mechanical appliances for propelling, applying, dispersing or spraying. In a preferably aspect, the method of administration of the invention allows spreading the pheromones to the animals closely enough (near the face of the mammals) so that the effects are both rapid and effective.

In ambient air the composition of pheromones preferably presents itself under a liquid form.

The device according to the invention may be, for example, a container, such as a bottle, with a propelling means, such as spraying means, and more specifically a air fluid spraying means. The device contains the composite foam of the invention. More specifically, the device presents the composite comprising the foam impregnated with the composition of pheromones. The device can be or comprise any mechanical appliances which create a stream of fluid (gas or liquid) passing through the composite foam impregnated with the composition of pheromones for propelling, dispersing, diffusing or spraying the composition of pheromones. A kit comprising a device may also be envisioned, the said kit comprising a device, in particular a container, the composite of the present invention, and optionally a booklet giving instructions to use said device. The said device may be in ready-to-use form, i.e. prefilled, or may need to be filled before use. In the latter case, the composition of pheromones may be in another device (such as a vial), to be poured into the device comprising the composite foam.

The present invention will be better understood in view of the Examples below.

### EXAMPLES

### Example 1

NP1 foam was impregnated with a pure pheromone composition without solvents (pheromonal composition A).

NP1 polyether polyurethane foam is a fully cross-linked reaction product of polyhydroxy polyol, isocyanates, catalysts, surfactants, colorants and water as described in the patent application WO2001074582.

**Table 1**

| | |
|---|---|
| Amount of the pheromonal composition A (by mg) | 100 mg |
| Amount of Foam NP1 (by mg) | 230 mg to 280 mg |

The pheromonal composition A was as follows:

**Table 2**

| Compounds | % by weight |
|---|---|
| Methyl linoleate | 21% |
| Methyl oleate | 35% |
| Methyl stearate | 13% |
| Methyl palmitate | 20% |
| Methyl pentadecanoate | 4% |
| Methyl myristate | 5% |
| Methyl laurate | 2% |

The obtained product works as Adaptil® spray (product sold by Ceva Santé Animale), and is presented in a device which is designed to be activated close to the dog's nose (around 20 cm) at the moment of transport to achieve an immediate (less than 5 minutes) calming effect upon the dog.

Product E: Device comprising NP1 foam impregnated with pheromonal composition A Product S (placebo): Device comprising NP1 foam without any pheromonal composition

### Test

49 dogs tested first without product (baseline), then tested twice after each treatment (Product S and Product E) according to a cross-over design

### Dogs profile:

- male or female dog, aged more than 18 months
- dog which regularly shows symptoms of stress or anxiety when transported by car
- dog which is currently healthy, and has not been diagnosed with a behaviour problem that has required treatment or behaviour modifications
- dog ever visits the vet
- dog who does not regularly use a calming product to minimise dog's stress or anxiety when travelling by car

### Test characteristic:

3 journeys in total (one baseline, two with either product test)
Dog travelled alone in car (i.e. with no other pets)
Product (S or E) was used close to the dog's nose 5 minutes before the trip
Car journey was between 30-minutes and an hour long
Journey did not conclude with a potentially stressful event for the dog (i.e. visiting the vet) No calming or anxiety-reducing products were used concurrently for the trip.

### Results

After each journey, dog owners had to score (from 1: no sign, to 10: maximum stress signs) their dog's anxiety for each of the following 10 signs (agitation, barking, cowering, panting, salivating, soiling, trembling, urination, vomiting, yawning).

For each stress sign, the performed analysis focused on the difference "baseline score" minus "score after travel". The "baseline score" is the observation of the ten symptoms done by the dog owners during travel without any behavior treatment.
2 scores are thus established:
- Sum 1 = scores of the differences for the 10 stress signs
- Sum 2 = scores of the differences for the 4 main stress signs (agitation, panting, salivating, trembling) mostly displayed during the baseline journey

**Table 3:**

| **Stress signs difference sum** | **Product E** | **Product S** | **P value** |
|---|---|---|---|
| 1 | 9.1±15.6 | 4.5±10.3 | 0.065 |
| 2 | 6.4±7.9 | 3.0±5.9 | 0.038 |

The product according to the invention (Product E) led to greater improvement of stress scores (higher difference from baseline) illustrating the main dogs stress signs, compared to stress scores changes observed when travelling with a placebo.

## Claims

1. A composite comprising a composition of pheromones and a composite foam, wherein the pheromones are selected from monocarboxylic and dicarboxylic acids with hydrocarbonated chains, saturated or unsaturated, linear or branched, liable to treat behavior or medical changes related to anxiety or stress of non-human mammals, and/or esters or salts of said acids, and the composite foam is a polyurethane that comprises both hydrophobic and hydrophilic polyurethane foam entities.

2. The composite according to anyone of claims 1, wherein the composite foam is impregnated with the composition of pheromones.

3. The composite according to anyone of claim 1 and 2, wherein pheromones are methyl esters of monocarboxylic and dicarboxylic acids.

4. The composite according to anyone of claim 1 and 2, wherein pheromones is a mixture comprising oleic acid, palmitic acid, azelaic acid, pimelic acid, capric acid, myristic acid, palmitoleic acid, linoleic acid, linolenic acid, stearic acid, valeric acid, arachidonic acid, lauric acid, n-butyric acid, isobutyric acid, [alpha]-methylbutyric acid, pivalic acid, γ-linoleic acid, eicosapentanoic acid, pentadecanonic acid, tridecanoic acid, docosahexanoic acid, or their esters, methyl esters, or salts .

5. The composite according to anyone of claims 1-4, for a use to prevent and / or treat at least one medical or behavioral stress problem of a non-human mammal.

6. The composite for a use according the claim 5, wherein the non-human mammal is a canine or feline.

7. The composite for a use according the claim 5, wherein the non-human mammal is a dog, and the medical or behavioral stress problem is more specifically a stress sign selected from excessive barking, agitation, trembling, shaking, shivering, drooling, salivating, cowering and hiding, licking, scratching, yawning, clinging to owners, trying to run away, attempting to escape, soiling the place, excessive pacing and panting, refusing to eat, vomiting, whining, urinating, defaecating, and any combination thereof.

8. The composite for a use according the claim 5, wherein the non-human mammal is a cat, and the medical or behavioral stress problem is more specifically a stress sign selected from scratching, urine marking, aggression, food intake disorders (anorexia or over-feeding), overgrooming (bald areas) or undergrooming (matted or soiled fur), house soiling, decreased levels of activity, appearing withdrawn (reduced desire to play or interact to owners), cystitis, a dermatological problem including excessive licking, alopecia, anorexia, obesity, diarrhea, infections tract, or any combination of said signs.

9. The composite for a use according to anyone of claims 5, 7 or 8, wherein the composition of pheromones is administered by propelling the composition onto, over or nearby the face of the non-human animal or a part thereof.

10. A device comprising a composite as defined in anyone of claims 1-4, and preferably means for the controlled release of the composition of pheromones into a stream of fluid passing through the composite foam containing the composition of pheromones.

11. The device according to claim 10, wherein said device is a container, preferably a bottle, with a air fluid spraying means.

12. A kit comprising a device as defined in claim 10 or 11, and optionally a booklet giving instructions to use said device.

## Patentansprüche

1. Ein Verbund umfassend eine Zusammensetzung von Pheromonen und einen Verbundschaum, wobei die Pheromone aus Mono- und Dicarbonsäuren mit linearen oder verzweigten, ungesättigten oder gesättigten Kohlenwasserstoffketten, die zur Behandlung von Verhaltensänderungen oder medizinischen Änderungen geeignet sind, die mit Angst oder Stress von nichthumanen Säugetieren verbunden sind, und/oder Estern oder Salzen besagter Säuren ausgewählt sind, und der Verbundschaum ein Polyurethan ist, das sowohl hydrophobe als auch hydrophile Polyurethanschaum-Einheiten umfasst.

2. Der Verbund gemäß Anspruch 1, wobei der Verbundschaum mit der Zusammensetzung von Pheromonen getränkt ist.

3. Der Verbund gemäß einem der Ansprüche 1 und 2, wobei die Pheromone Methylester von Mono- und Dicarbonsäuren sind.

4. Der Verbund gemäß einem der Ansprüche 1 und 2, wobei die Pheromone ein Gemisch sind, umfassend Oleinsäure, Palmitinsäure, Azelainsäure, Pimelinsäure, Caprinsäure, Myristinsäure, Palmitoleinsäure, Linolsäure, Linolensäure, Stearinsäure, Valeriansäure, Arachidonsäure, Laurinsäure, n-Buttersäure, Isobuttersäure, [alpha]-Methylbuttersäure, Pivalinsäure, y-Linolsäure, Eicosapentansäure, Pentadecansäure, Tridecansäure, Docosahexansäure oder ihre Ester, Methylester oder Salze.

5. Der Verbund gemäß einem der Ansprüche 1 bis 4 für eine Verwendung zur Prävention und/oder Behandlung wenigstens eines medizinischen oder verhaltensbedingten Stressproblems eines nichthumanen Säugetiers.

6. Der Verbund für eine Verwendung gemäß Anspruch 5, wobei das nichthumane Säugetier ein hundeartiges oder katzenartiges Tier ist.

7. Der Verbund für eine Verwendung gemäß Anspruch 5, wobei das nichthumane Säugetier ein Hund ist und das medizinische oder verhaltensbedingte Stressproblem spezifischer ein Stressanzeichen ist, ausgewählt aus übermäßigem Bellen, Unruhe, Zittern, Schütteln, Frösteln, Sabbern, Speichelfluss, Kauern und Verstecken, Lecken, Kratzen, Jaulen, Klammern an den Besitzer, Weglaufversuchen, Fluchtversuchen, Verschmutzen des Orts, übermäßigem Auf- und Abgehen und Hecheln, Verweigerung der Nahrungsaufnahme, Erbrechen, Winseln, Urinieren, Koten und einer beliebigen Kombination davon.

8. Der Verbund für eine Verwendung gemäß Anspruch 5, wobei das nichthumane Säugetier eine Katze ist und das medizinische oder verhaltensbedingte Stressproblem spezifischer ein Stressanzeichen ist, ausgewählt aus Kratzen, Harnmarkieren, Aggression, Nahrungsaufnahmestörungen (Anorexie oder Überfütterung), Überpflege (kahle Stellen) oder ungenügender Pflege (verfilztes oder verschmutztes Fell), Hausverschmutzen, verminderter Aktivität, Auftreten von Zurückgezogenheit (verminderter Spiel- oder Interaktionswunsch mit Besitzer), Zystitis, einem Hautproblem, einschließlich übermäßigen Leckens, Alopezie, Anorexie, Obesität, Diarrhö, Traktinfektionen oder einer beliebigen Kombination besagter Anzeichen.

9. Der Verbund für eine Verwendung gemäß einem der Ansprüche 5, 7 oder 8, wobei die Zusammensetzung von Pheromonen durch Verwirbeln der Zusammensetzung auf, über oder nahe dem Gesicht des nichthumanen Säugetiers oder einem Teil davon verabreicht wird.

10. Eine Vorrichtung umfassend einen Verbund wie in einem der Ansprüche 1 bis 4 definiert, und vorzugsweise Mittel für die kontrollierte Freisetzung der Zusammensetzung von Pheromonen in einen Flüssigkeitsstrom, der durch den Verbundschaum strömt, der die Zusammensetzung von Pheromonen enthält.

11. Die Vorrichtung gemäß Anspruch 10, wobei besagte Vorrichtung ein Behälter, vorzugsweise eine Flasche, mit einem Luft-Flüssigkeits-Sprüh-Mittel ist.

12. Ein Kit umfassend eine Vorrichtung, wie in Anspruch 10 oder 11 definiert, und gegebenenfalls eine Broschüre mit Anweisungen für den Gebrauch besagter Vorrichtung.

## Revendications

1. Composite comprenant une composition de phéromones et une mousse composite, dans lequel les phéromones sont choisies parmi les acides monocarboxyliques et dicarboxyliques à chaîne hydrocarbonée, saturés ou insaturés, linéaires ou ramifiés, capable de traiter les changements comportementaux ou les problèmes médicaux liés à l'anxiété ou au stress de mammifères non humains, et/ou esters ou sels desdits acides, et la mousse composite est un polyuréthane qui comprend à la fois des entités de mousse polyuréthane hydrophobes et hydrophiles.

2. Composite selon la revendication 1, dans lequel la mousse du composite est imprégnée avec la composition de phéromones.

3. Composite selon l'une quelconque des revendications 1 à 2, dans lequel les phéromones sont des esters de méthyle d'acides monocarboxyliques et dicarboxyliques.

4. Composite selon l'une quelconque des revendications 1 à 2, dans lequel les phéromones sont un mélange comprenant de l'acide oléique, acide palmitique, acide azélaïque, acide pimélique, acide caprique, acide myristique, acide palmitoléique, acide linoléique, acide linolénique, acide stéarique, acide valérique, acide arachidonique, acide laurique, acide n-butyrique, acide isobutyrique, acide [alpha]-méthylbutyrique, acide pivalique, acide γ-linoléique, acide eicosapentanoïque, acide pentadécanoïque, acide tridécanoïque, acide docohexanoïque, ou leurs esters, esters de méthyle, ou sels.

5. Composite selon l'une quelconque des revendications 1 à 4, destiné à être utilisé pour prévenir et/ou traiter au moins trouble de stress lié à un problème médical ou comportemental chez un mammifère non humain.

6. Composite pour son utilisation selon la revendication 5, où le mammifère non humain est de race canine ou féline.

7. Composite pour son utilisation selon la revendication 5, où le mammifère non humain est un chien, et le trouble de stress lié à un problème médical ou comportemental est plus spécifiquement un signe de stress choisi parmi les aboiements excessifs, l'agitation, les tremblements, les tressautements, les frissons, la salivation, l'hypersalivation, la propension à se recroqueviller et à se cacher, l'action de se lécher, de se gratter, de bailler, de s'accrocher à ses maîtres, d'essayer de fuir, de s'échapper, la propension à souiller l'endroit, à tourner en rond et à haleter de manière excessive, le refus de s'alimenter, les vomissements, les gémissements, le besoin d'uriner, de déféquer, et toute combinaison de ceux-ci.

8. Composite pour son utilisation selon la revendication 5, où le mammifère non humain est un chat, et le trouble de stress lié à un problème médical ou comportemental est plus spécifiquement un signe de stress choisi parmi la propension à se gratter, le marquage urinaire, l'aggression, les troubles alimentaires (anorexie ou boulimie), le toilettage compulsif (zones alopéciques) ou insuffisant (fourrure mate ou souillée), la propension à souiller la maison, des niveaux d'activité en baisse, avec propension au renfermement (envie réduite de jeu et d'interaction avec ses maîtres), la cystite, un problème dermatologique comprenant un léchage excessif, l'alopécie, l'anorexie, l'obésité, la diarrhée, les infections, ou toute combinaison de ceux-ci.

9. Composite pour son utilisation selon l'une quelconque des revendications 5, 7 ou 8, où la composition de phéromones est administrée par pulvérisation de la composition sur, au-dessus ou à proximité de la tête de l'animal non humain, ou d'une partie de celle-ci.

10. Dispositif comprenant un composite tel que défini dans l'une quelconque des revendications 1 à 4, et de préférence un moyen de libération contrôlée de la composition de phéromones dans un flux de fluide traversant la mousse composite contenant la composition de phéromones.

11. Dispositif selon la revendication 10, dans lequel ledit dispositif est un récipient, de préférence, un flacon, pourvu d'un moyen de pulvérisation de fluide.

12. Kit comprenant un dispositif tel que défini dans la revendication 10 ou 11, et éventuellement un livret contenant les instructions d'utilisation dudit dispositif.
